# EUROPEAN PATENT APPLICATION

(11) **EP 2 246 085 A1**
(43) Date of publication of application: **03.11.2010**
(21) Application number: 09251194.8
(22) Date of filing: 28.04.2009
(51) Int. Cl.: A61M 5/32, A61M 5/28, A61M 5/315

(54) **Disposable syringe with built-in carpule**

(71) Applicant: Shue, Ming-Jeng, Hsi District, Taichung City (TW); Huang, Deborah, Taichung City (TW); Shue, Phillip, Hsi District, Taichung City (TW)
(72) Inventor: Shue, Ming-Jeng, Hsi District, Taichung City (TW); Huang, Deborah, Taichung City (TW); Shue, Phillip, Hsi District, Taichung City (TW)
(74) Representative: Robertson, James Alexander

(57) **Abstract**

A disposable syringe includes a pre-filled carpule (3) which is retained in a barrel (1) by means of a lunge member (22), a ring grip (21) and a carrier unit (34). A retaining mechanism (13) is disposed to guard the carrier unit (34) against movement away from a locked position. A needle assembly (33,51,37) is connected to the carpule (3). A plunger (4) is engaged with a seal member (32) of the carpule (3) to cooperate with a tubular wall (31) to define a sealed chamber (31a) that is communicated with the needle assembly (33,51,37). After the plunger (4) is turned relative to the barrel (1) to move the carrier unit (34) to the unlocked position, the carpule (3) can be pressed forwardly to force the carrier unit (34) to release the ring grip (21) from the lunge member (22) such that the needle assembly (33,51,37) and the carpule (3) are biased by a biasing member (23) rearwardly to retract the needle cannula (33) into the barrel (1).

## Description

This invention relates to a disposable syringe, more particularly to a disposable syringe with a carpule.

In U.S. Patent No. 6, 221, 055 B1 for a retractable dental syringe, there is disclosed a pre-filled syringe, which includes a barrel, a carpule and plunger assembly that is filled with medication and that is received in the barrel from a rear open end of the barrel and that is communicated with a needle when punctured by a rear tip end of the needle, and a push ring for holding the needle in the barrel. At the end of an injection, the plunger is pressed to move the carpule forwardly to remove the push ring so as to permit retraction of the needle. However, the carpule cannot be accommodated in the barrel in advance, and the carpule and the barrel have to be packed separately for subsequent assembly in use, thereby resulting in increased operation time. In addition, such structure complicates the manufacture of the syringe.

Therefore, in U.S. Patent Application No. 11/601,526, the applicants disclosed a disposable syringe with a built-in carpule which includes a carpule that can be pre-assembled in a barrel so that clinical injection operation by health care workers is simplified and is convenient to conduct. Moreover, the syringe can be conveniently rendered unreusable and the needle retraction is smooth after injection in a single operation.

It is desirable to improve the aforesaid syringe to ensure firm retention of a pre-filled and sealed carpule inserted into a barrel before use, and stabler and smoother operation.

An object of the present invention is to provide a disposable syringe with a built-in carpule which ensures firm retention of a pre-filled and sealed carpule in a barrel before use, and which can be operated more stably and smoothly.

According to this invention, the disposable syringe with a built-in carpule includes a barrel which has a surrounding barrel wall defining front and rear compartments, and an insertion opening communicated with the front compartment. A holding member is disposed in the insertion opening, extends rearwardly to terminate at a limit end, and is spaced apart from a front wall portion of the barrel wall to define an accommodation space therebetween. A lunge member has a front lunge end which abuts against the limit end, and which has a resting surface confronting the accommodation space, and a ring head which has an annular gripped surface that is spaced apart from the front wall portion to define a surrounding clearance therebetween. A ring grip is disposed in the surrounding clearance, and has inner and outer annular gripping surfaces to retainingly engage the gripped surface and the front wall portion, respectively. A seat unit is configured to secure a communicating end of a needle cannula therein. A carpule includes a tubular wall and a seal member. The tubular wall is disposed to be rotatable in the rear compartment, and extends forwardly to terminate at a front tubular edge surface that extends radially to be integrally formed with a distal hub segment of the seat unit. The seal member is disposed to be in fluid-tight engagement with the tubular wall, and is spaced apart from the front tubular edge surface to define, in cooperation with the tubular wall, a sealed chamber for holding liquid medicament. The seal member is movable forwardly to expel the liquid medicament out of the sealed chamber to the front tubular edge surface, thereby placing the carpule in a used-up position. A carrier unit is rotatable with the carpule between locked and unlocked positions by an external twisting force, and includes a push stud which is pushed forward once the carrier unit is in the unlocked position, so as to move the ring grip forward so as to release the front lunge end from the limit end, thereby placing the lunge member in a released position. A retaining mechanism is disposed to guard the carrier unit against movement away from the locked position. A key-and-keyway mechanism is disposed to guide the tubular wall of the carpule to move along the barrel wall once the carrier unit is displaced to the unlocked position. A plunger has a push end which moves the seal member forwardly along the tubular wall of the carpule, and an operating end which is operable to turn the tubular wall together with the carrier unit to the unlocked position. A biasing member is disposed in the accommodation space to abut against the resting surface such that, once the lunge member is placed in the released position, the biasing member biases the ring head so as to force the distal hub segment and the carpule towards the rear barrel end to retract the needle cannula into the barrel, thereby placing the needle cannula in a disposal position.

Other features and advantages of the present invention will become apparent in the following detailed description of the preferred embodiments of the invention, with reference to the accompanying drawings, in which:
Fig. 1 is an exploded sectional view of the first preferred embodiment of a disposable syringe according to this invention;
Fig. 2 is a sectional view of the first preferred embodiment in an initial assembled state;
Fig. 3 is a cross-sectional view taken along line III-III of Fig. 2, showing a carrier unit in a locked position;
Fig. 4 is a sectional view of the first preferred embodiment during an injection stroke;
Fig. 5 is a cross-sectional view similar to Fig. 3, but showing the carrier unit in an unlocked position;
Fig. 6 is a fragmentary sectional view of the first preferred embodiment after completion of the injection stroke;
Fig. 7 is a sectional view of the first preferred embodiment, showing a lunge member in a state of release from a ring grip;
Fig. 8 is a sectional view of the first preferred embodiment, showing a needle cannula fully retracted into a barrel;
Fig. 9 is a sectional view of the second preferred embodiment of a disposable syringe according to this invention;
Fig. 10 is a sectional view of the second preferred embodiment in a state ready for use;
Fig. 11 is a sectional view of the second preferred embodiment, showing a needle cannula fully retracted into a barrel;
Fig. 12 is a sectional view of the third preferred embodiment of a disposable syringe according to this invention in an initial assembled state;
Fig. 13 is a sectional view of the third preferred embodiment after completion of an injection stroke;
Fig. 14 is a sectional view of the fourth preferred embodiment of a disposable syringe according to this invention in an initial assembled state;
Fig. 14 (A) is a cross-sectional view taken along line (I)-(I) of Fig. 14;
Fig. 15 is a sectional view of the fourth preferred embodiment in a state of use;
Fig. 16 is a sectional view of the fifth preferred embodiment of a disposable syringe according to this invention in an initial assembled state;
Fig. 17 is a sectional view of the fifth preferred embodiment, showing a needle cannula fully retracted into a barrel;
Fig. 18 is a sectional view of the sixth preferred embodiment of a disposable syringe according to this invention in an initial assembled state;
Fig. 19 is a sectional view of the sixth preferred embodiment, showing a needle cannula fully retracted into a barrel;
Figs. 20 to 22 are, respectively, sectional views of the seventh, eighth and ninth preferred embodiments of a disposable syringe according to this invention in an initial assembled state;
Fig. 23 is a sectional view of the tenth preferred embodiment of a disposable syringe according to this invention in an initial assembled state;
Fig. 24 is a sectional view of the tenth preferred embodiment, showing a needle cannula fully retracted into a barrel; and
Fig. 25 is a sectional view of the eleventh preferred embodiment of a disposable syringe according to this invention, showing a needle cannula fully retracted into a barrel.

Before the present invention is described in greater detail, it should be noted that same reference numerals have been used to denote like elements throughout the specification.

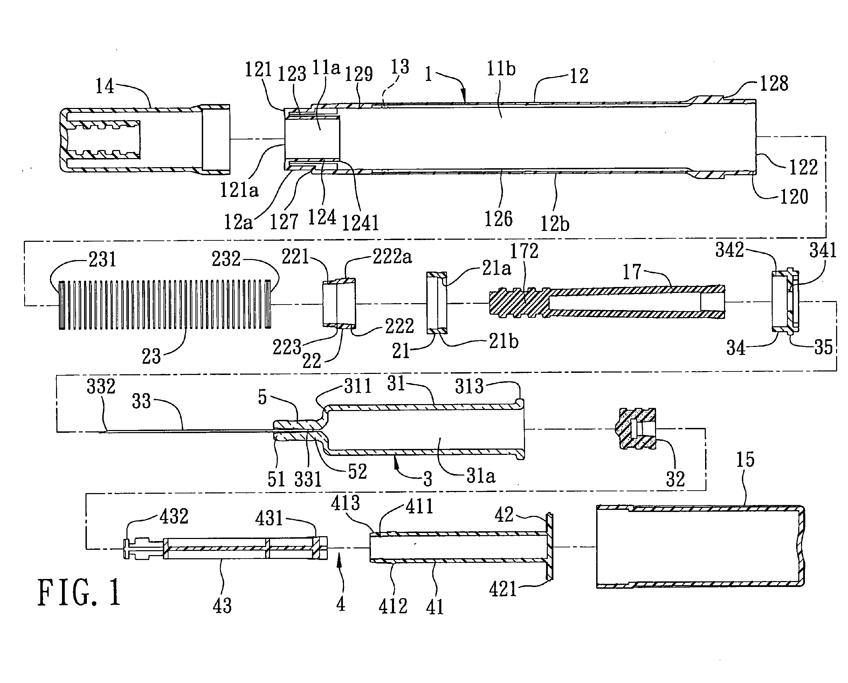

Referring to Figs. 1 to 4, the first preferred embodiment of a disposable syringe according to the present invention is shown to comprise a barrel 1, a lunge member 22, a ring grip 21, a needle cannula 33, a seat unit 5, a carrier unit 34, a carpule 3, an air-tight sleeve 17, a key-and-keyway mechanism 35,126, a retaining mechanism 13, a plunger 4, front and rear protectors 14,15, and a biasing member 23.

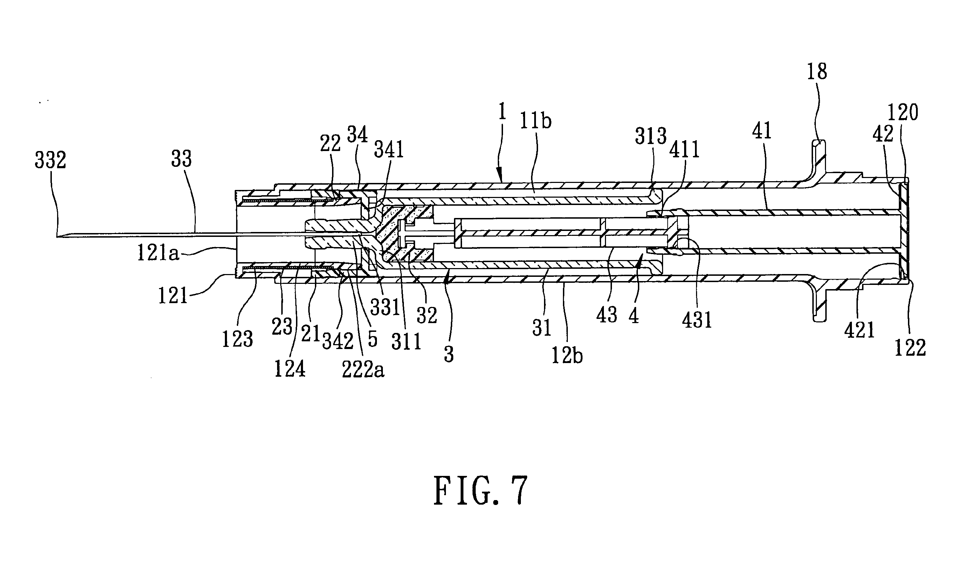

The barrel 1 includes front and rear barrel ends 121,122 opposite to each other along an axis in an axial direction, a surrounding barrel wall 12 which interconnects the front and rear barrel ends 121,122, and which includes front and rear wall portions (12a,12b) that are disposed proximate to the front and rear barrel ends 121,122, respectively, and that define front and rear compartments (11a,11b), respectively, and a finger flange 18 (as shown in Fig. 7) which is disposed proximate to the rear barrel end 122. The front barrel end 121 defines an insertion opening (121a) which extends in the axial direction to communicate with the front compartment (11a). A holding member 124 is in the form of an annular wall which is disposed in the insertion opening (121a), which extends rearwardly from the front barrel end 121 to terminate at a limit end 1241, and which is spaced apart from the front wall portion (12a) radially to define an accommodation space 123 therebetween. The front and rear wall portions (12a,12b) respectively have front and rear shoulder abutments 127,128 which are respectively disposed adjacent to the front and rear barrel ends 121,122 and which respectively face forwardly and rearwardly. An engaging ring 129 is formed on an inner surface of the front wall portion (12a) adjacent to the holding member 124. A retaining portion 120 in the form of an annular groove 120 is formed in an inner surface of the rear wall portion (12b) adjacent to the rear barrel end 122.

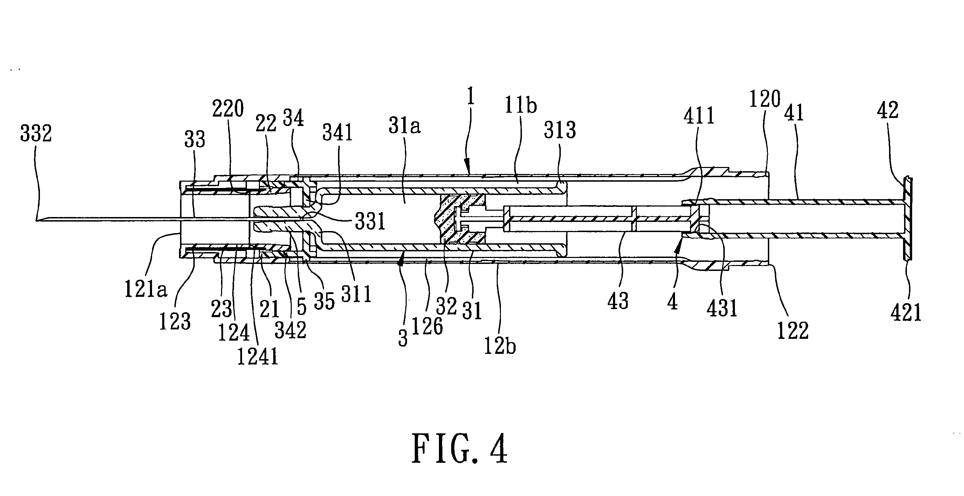

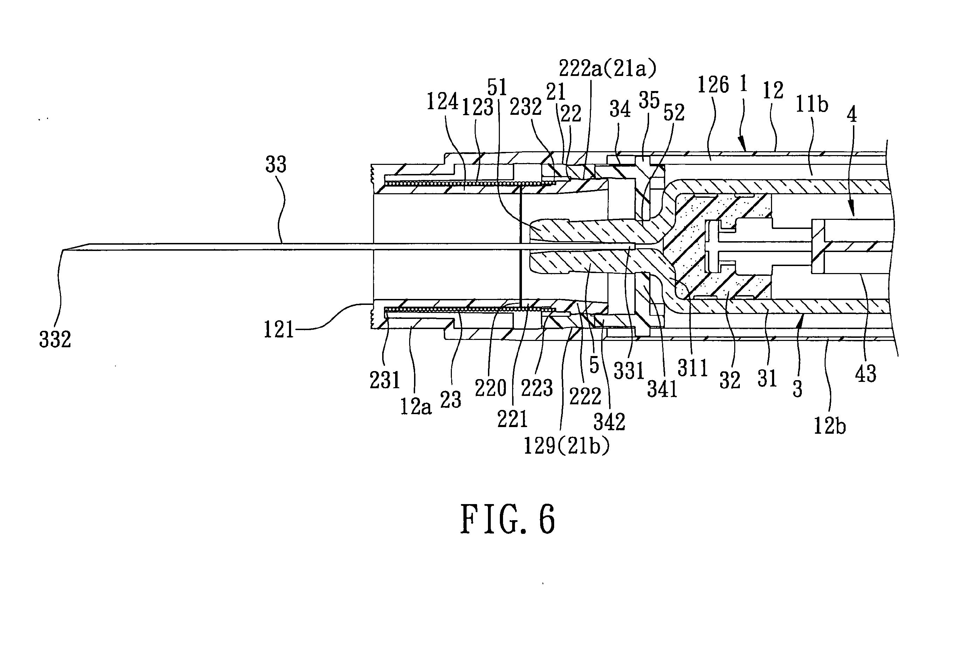

The lunge member 22 has a front lunge end 221 which is disposed to confront and which is spaced apart from the limit end 1241 in the axial direction by a small clearance 220 in a position of use (as shown in Figs. 4 and 6) to permit abutting engagement with the limit end 1241, and which has a resting surface 223 confronting the accommodation space 123, and a ring head 222 opposite to the front lunge end 221 in the axial direction. The ring head 222 has an annular gripped surface (222a) that is spaced apart from the front wall portion (12a) to define a surrounding clearance therebetween.

The ring grip 21 is squeezed in the surrounding clearance between the annular gripped surface (222a) and the front wall portion (12a) to be retained to the engaging ring 129, and has an inner annular gripping surface (21a) and an outer annular gripping surface (21b) to retainingly engage the gripped surface (222a) and the front wall portion (12a), respectively, by virtue of a first friction force such that, in the position of use, the first friction force is of such a magnitude as to guard the front lunge end 221 against movement away from the limit end 1241. Specifically, the ring head 222 is made from a flexible material such that, as a result of squeezing of the ring grip 21 in the surrounding clearance, the ring head 222 acquires an urging force so as to generate the first friction force between the annular gripped surface (222a) and the inner gripping surface (21a), and between the outer annular gripping surface (21b) and the front wall portion (12a).

The needle cannula 33 has a tip end 332 which is disposed forwardly of the front barrel end 121 in the position of use, and a communicating end 331 which is opposite to the tip end 332 along the axis.

The seat unit 5 is configured to secure the communicating end 331 therein, and has proximate and distal hub segments 51,52 relative to the tip end 332. In this embodiment, the proximate and distal hub segments 51, 52 are integrally formed with each other.

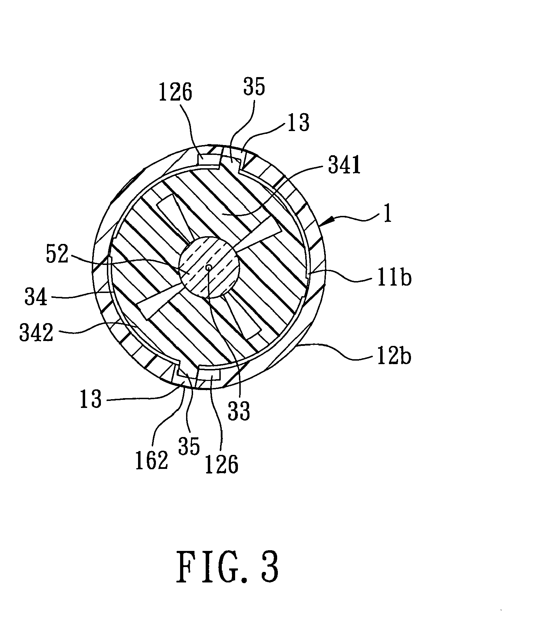

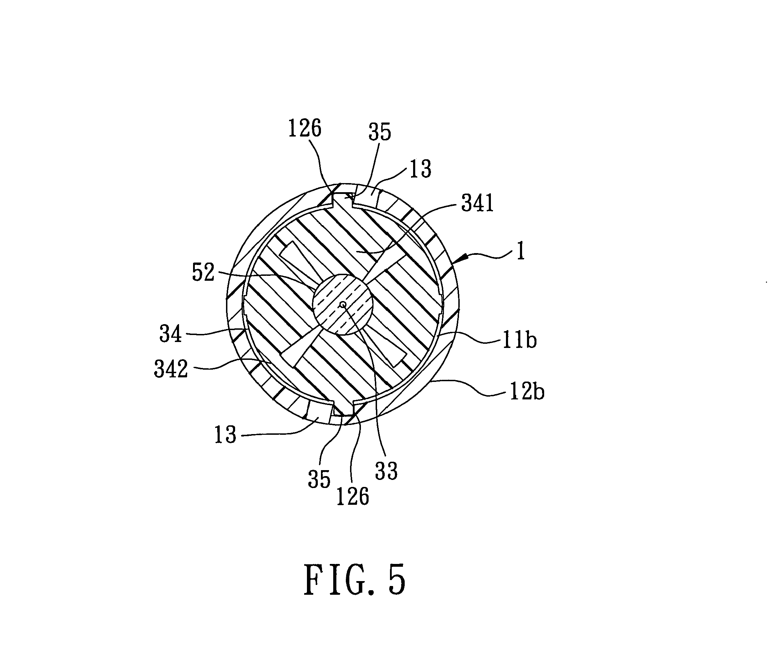

The carrier unit 34 is disposed to be turnable about the axis between locked and unlocked positions (as shown in Figs. 3 and 5), and includes a web 341 which is press-fitted to and which extends radially from the distal hub segment 52 and towards the surrounding barrel wall 12 to terminate at a peripheral portion, and a plurality of push studs 342 which are formed with the peripheral portion of the web 341 and which are frictionally engaged with and which are permitted to rotate relative to the ring head 222. Thus, once the carrier unit 34 is in the unlocked position, the push studs 342 are permitted to be pushed forward by a pushing force to move the ring grip 21 forward against the first friction force so as to release the front lunge end 221 from the limit end 1241, thereby placing the lunge member 22 in a released position (as shown in Fig. 7). It is noted that the push studs 342 may be formed integrally as a single annular piece.

The carpule 3 includes a glass-made tubular wall 31 and a seal member 32. The tubular wall 31 is configured to be rotatable in the rear compartment (11b) about the axis, extends forwardly to terminate at a front tubular edge surface 311 that extends radially to be integrally formed with the distal hub segment 52 of the seat unit 5, and has a rear annular flange 313 opposite to the front tubular edge surface 311. The tubular wall 31 is in a frictional and slidable engagement with the rear wall portion (12b). In this embodiment, the tubular wall 31 is made from a glass material. The seal member 32 is disposed to be in fluid-tight engagement with the tubular wall 31 by virtue of a second friction force which is larger than an external twisting force such that the carrier unit 34 is permitted to be turned from the locked position to the unlocked position by the external twisting force. The seal member 32 is spaced apart from the front tubular edge surface 311 in the axial direction to define, in cooperation with the tubular wall 31, a sealed chamber (31a) for holding liquid medicament, and is movable forwardly to expel the liquid medicament out of the sealed chamber (31a) to the front tubular edge surface 311, thereby placing the carpule 3 in a used-up position (as shown in Fig. 6).

The air-tight sleeve 17 is removably sleeved on the seat unit 5 to shield the needle cannula 33, and has an elastomeric front end 172 such that the tip end 332 of the needle cannula 33 is trapped in the elastomeric front end 172 for keeping air-tightness of the sealed chamber (31a).

The key-and-keyway mechanism 35,126 includes a pair of pegs 35 which extend radially from the peripheral portion of the web 341, and a pair of guide slots 126 which are formed in the rear wall portion (12b) and which extend in the axial direction for sliding movement of the pegs 35, respectively, in the axial direction once the carrier unit 34 is displaced to the unlocked position, as shown in Fig. 5.

The retaining mechanism includes a pair of retaining grooves 13 which extend transversely to and which are communicated with front ends of the guide slots 126, respectively, and which are disposed for retaining the pegs 35 therein so as to place the carrier unit 34 in the locked position, as shown in Fig. 3. An annular sealing strip 162 is attached to the rear wall portion (12b) to seal the retaining grooves 13.

The plunger 4 includes first and second plunger bodies 43,41. The first plunger body 43 has a push end 432 which is engaged with the seal member 32 and which moves with the seal member 32 forwardly along the tubular wall 31 against the second friction force so as to bring the carpule 3 to the used-up position, and a first rear end portion 431 opposite to the push end 432. The second plunger body 41 has an operating end 42 which extends outwardly of the rear barrel end 122 to be subjected to the external twisting force to turn the tubular wall 31 of the carpule 3 together with the carrier unit 34 to the unlocked position, and a second front end portion 413 which is opposite to the operating end 42. The second front end portion 413 has an inner flange 411 which is releasably retained with the first rear end portion 431 by a third friction force that is greater than the second friction force so as to permit the seal member 32 to be moved forwardly to thereby place the carpule 3 in the used-up position, and that is greater than the first friction force such that, after the push studs 342 are pushed forward to place the lunge member 22 in the released position, the second plunger body 41 is permitted to move relative to the first plunger body 43, thereby permitting the seat unit 5 and the carpule 3 to be retracted rearwardly to the disposal position. In addition, the second plunger body 41 has an abutment 412 which is disposed on the second front end portion 413.

The front protector 14 is detachably sleeved on the front wall portion (12a) and abuts against the front shoulder abutment 127 for shielding the needle cannula 33. The rear protector 15 is detachably sleeved on the rear wall portion (12b), and abuts against the rear shoulder abutment 128 for shielding the plunger 4. Preferably, the front protector 14 is threadedly engaged with the elastomeric front end 172 of the air-tight sleeve 17. In order to obtain a good sealing effect, front and rear sealing strips 161 are disposed to peelably adhere the front protector 14 to the front wall portion (12a), and to peelably adhere the rear protector 15 to the rear wall portion (12b), respectively.

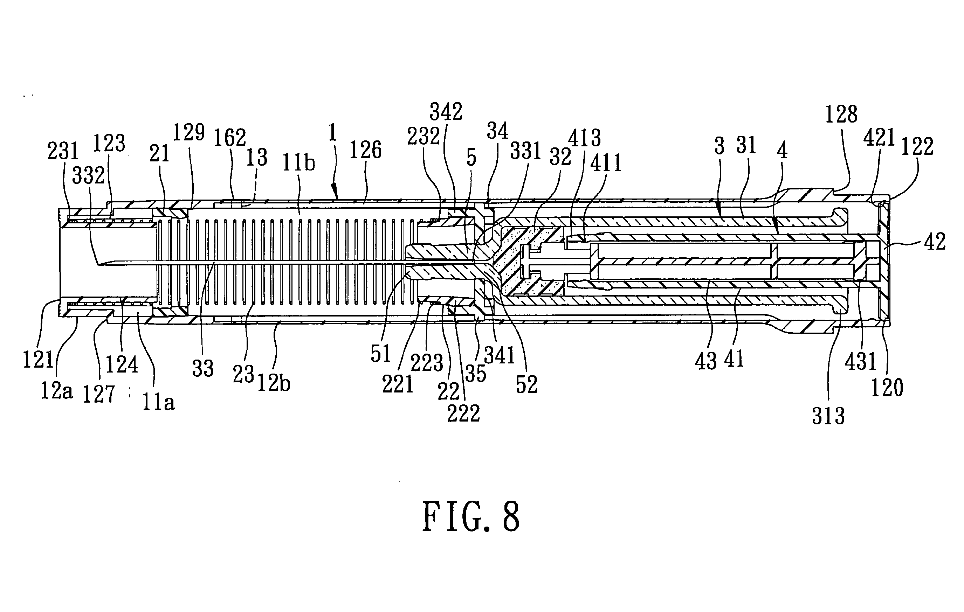

The biasing member 23 is disposed in the accommodation space 123, and has two opposite ends 231,232 which abut against the front barrel end 121 and the resting surface 223. Thus, once the lunge member 22 is placed in the released position, the biasing member 23 biases the ring head 222 to force the seat unit 5 and the carpule 3 towards the rear barrel end 122 so as to retract the tip end 332 of the needle cannula 33 into the barrel 1, thereby placing the needle cannula 33 in a disposal position, as shown in Fig. 8.

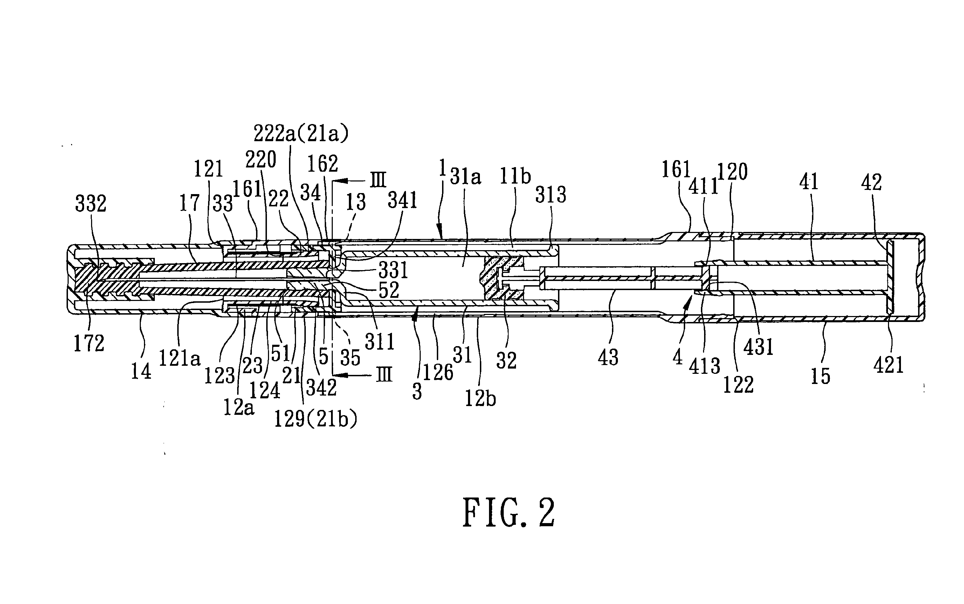

Referring to Figs. 2 and 4, in the injection stroke, the front and rear protectors 14,15 are removed first to expose the needle cannula 33 and the plunger 4. The operator holds the finger flange 18 (see Fig. 7) with his/her index and middle fingers and presses the operating end 42 of the plunger 4 forwardly with his/her thumb such that the seal member 32 is moved forwardly along the tubular wall 31 of the carpule 3 so as to expel air from the sealed chamber (31a) and to adjust the amount of the medicament as required. Due to the second friction force generated between the seal member 32 and the tubular wall 31, the operator can hold the barrel 1 with one hand, and apply an external twisting force to the plunger 4 to turn the carrier unit 34 to the unlocked position so as to disengage the pegs 35 from the retaining grooves 13. Subsequently, the pushing force is applied to the operating end 42 to introduce the medicament into a recipient of the patient through the needle cannula 33 and to place the carpule 3 in the used-up position, thereby completing the injection stroke, as shown in Fig. 6.

With reference to Figs. 5 to 7, the external pushing force is subsequently applied to the operating end 42 to force the push studs 342 to move the ring grip 21 forwardly. The lunge member 22 is also moved forwardly to abut against the limit end 1241. The clearance 220 can serve as a triggering space 220 to permit a slight forward movement of the lunge member 22 and the ring grip 21 for diminishing the friction resistance between the ring grip 21 and the lunge member 22, and between the ring grip 21 and the front wall portion (12a), thereby facilitating a subsequent forward movement of the grip ring 21. Thereafter, since the lunge member 22 is blocked by the limit end 1241, the pushing force applied to the push studs 342 forces the ring grip 21 to disengage from the lunge member 22, thereby placing the lunge member 22 in the released position. At the same time, the push studs 342 are moved to be in frictional engagement with the gripped surface (222a) of the ring head 222 until the web 341 is blocked by the lunge member 22. Further, the second plunger body 41 is moved forwardly relative to the first plunger body 43 against the third friction force until a periphery 421 of the operating end 42 is retained by the retaining portion 120 of the barrel 1, thereby guarding against a rearward movement of the second plunger body 41 for preventing reuse of the syringe.

As shown in Fig. 8, once the lunge member 22 is placed in the released position, the biasing force of the biasing member 23 is released to bias the ring head 222 to lunge at the web 341, which, together with the seat unit 5 and the carpule 3, are moved towards the rear barrel end 122 so as to retract the tip end 332 of the needle cannula 33 into the front compartment (11a), thereby placing the needle cannula 33 in the disposal position. At the same time, the first plunger body 43 is fully retracted into the second plunger body 41.

It is noted that the shape and size of the component parts of the disposable syringe described above may be varied. For example, the barrel 1, the needle cannula 33, the carpule 3, and the plunger 4 may be made longer to render the syringe suitable for performing a deep injection.

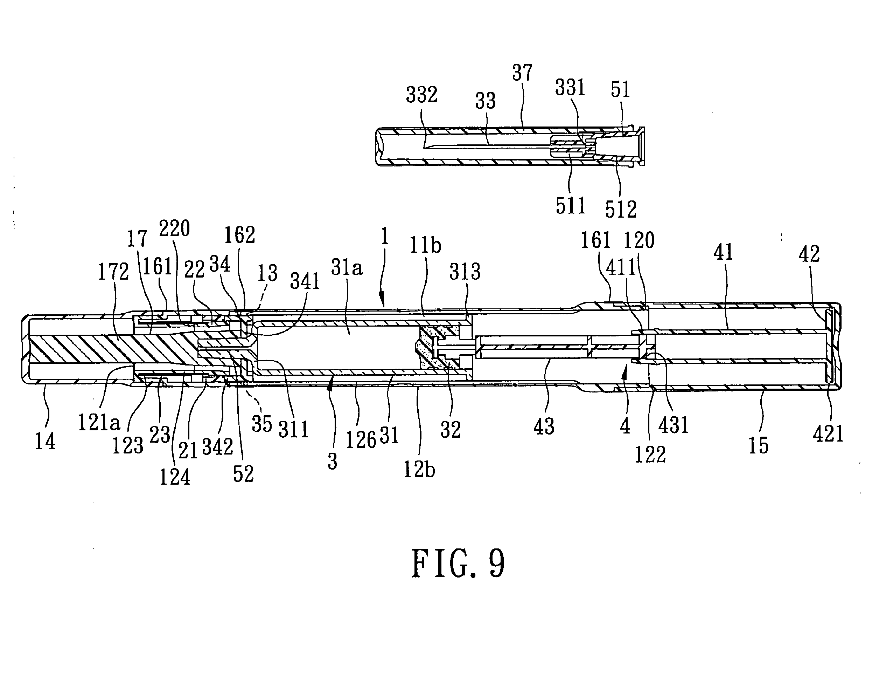

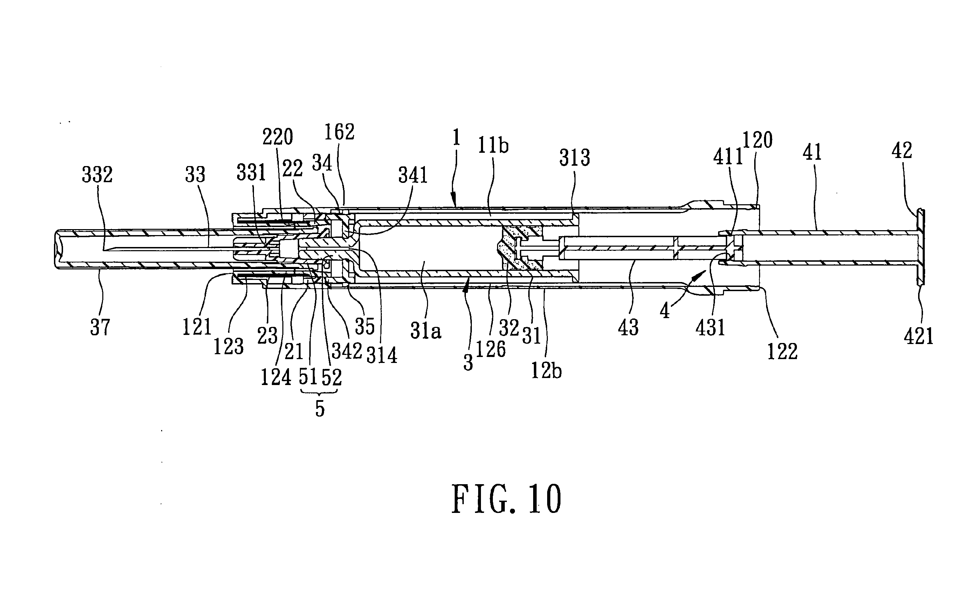

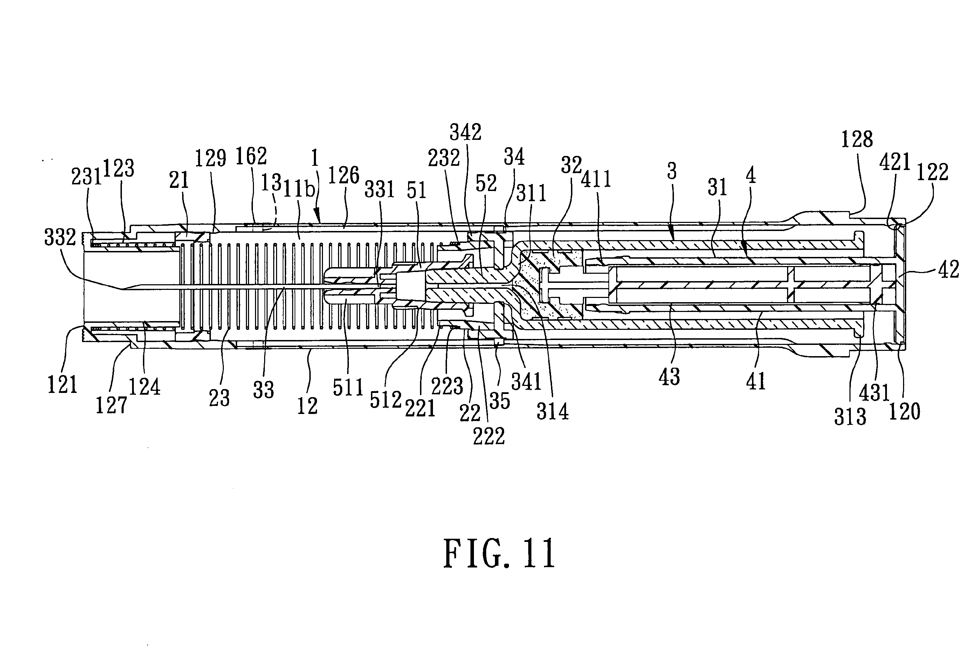

Referring to Figs. 9 to 11, the second preferred embodiment of a disposable syringe according to this invention is shown to be similar to the previous embodiment in construction. In the second embodiment, the proximate hub segment 51 of the seat unit 5 has a securing end 511 in which the communicating end 331 of the needle cannula 33 is secured, and a sleeve end 512 which extends from the securing end 511 in the axial direction, and which is detachably sleeved on the distal hub segment 52 so as to communicate the communicating end 331 with the sealed chamber

(31a). In addition, a tip protector 37 is sleeved on the proximate hub segment 51 to cover the needle cannula 33. Thus, the needle cannula 33, the proximate hub segment 51 and the tip protector 37 are assembled as a needle assembly. The air-tight sleeve 17 is sleeved on the distal hub segment 52 to maintain the air-tightness of the sealed chamber (31a) before use.

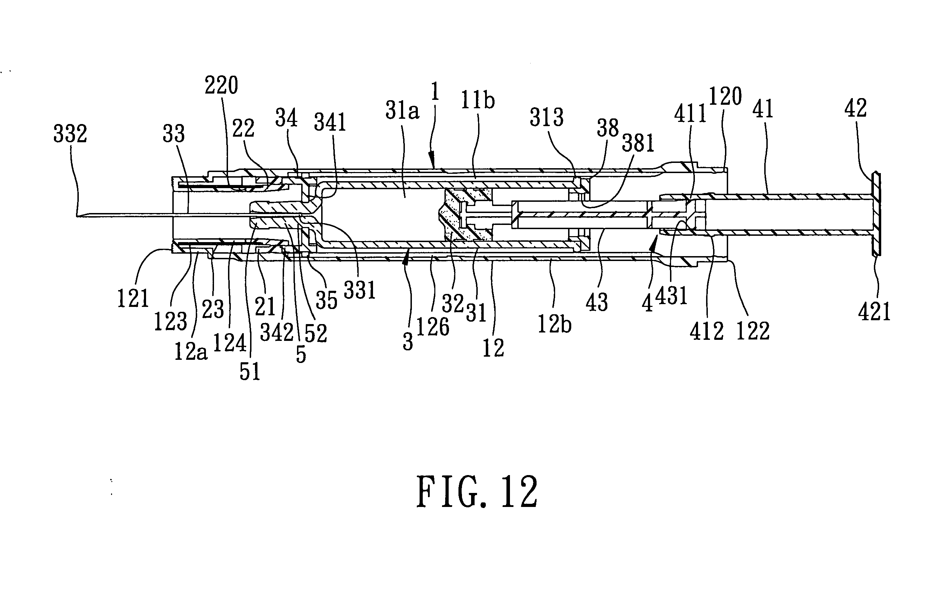

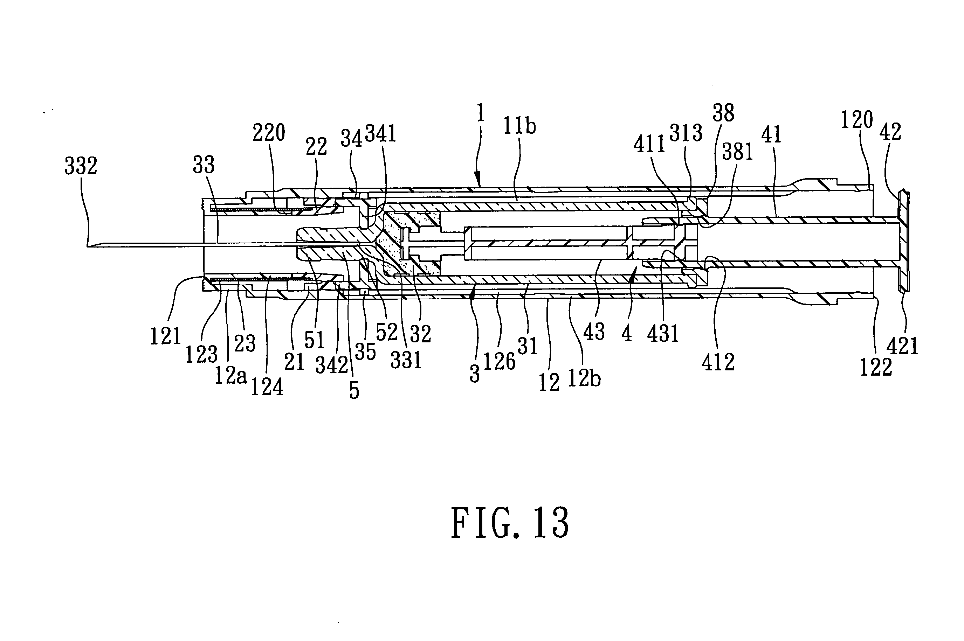

Referring to Figs. 12 and 13, the third preferred embodiment of a disposable syringe according to this invention is shown to be similar to the first embodiment in construction. In the third embodiment, an annular gasket 38, made from a plastic material, is disposed on the rear annular flange 313 of the tubular wall 31, and has an annular shoulder 381 which extends radially and inwardly. Hence, when the carpule 3 is in the used-up position, the abutment 412 of the plunger 4 abuts against the annular shoulder 381 so as to impart the pushing force from the plunger 4 to the carpule 3.

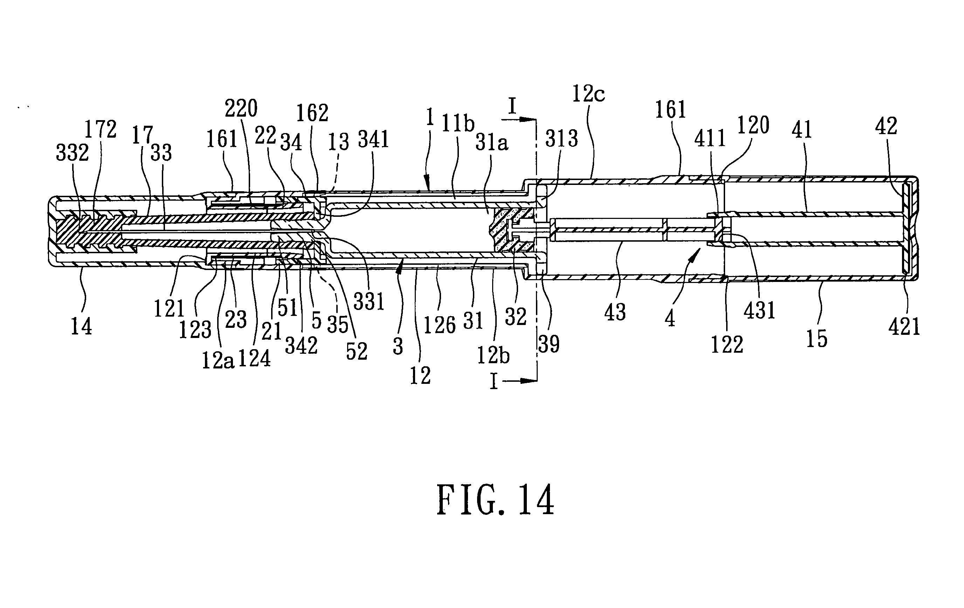

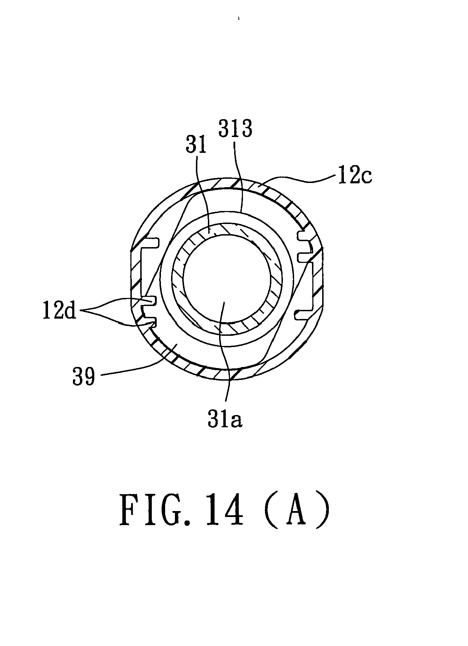

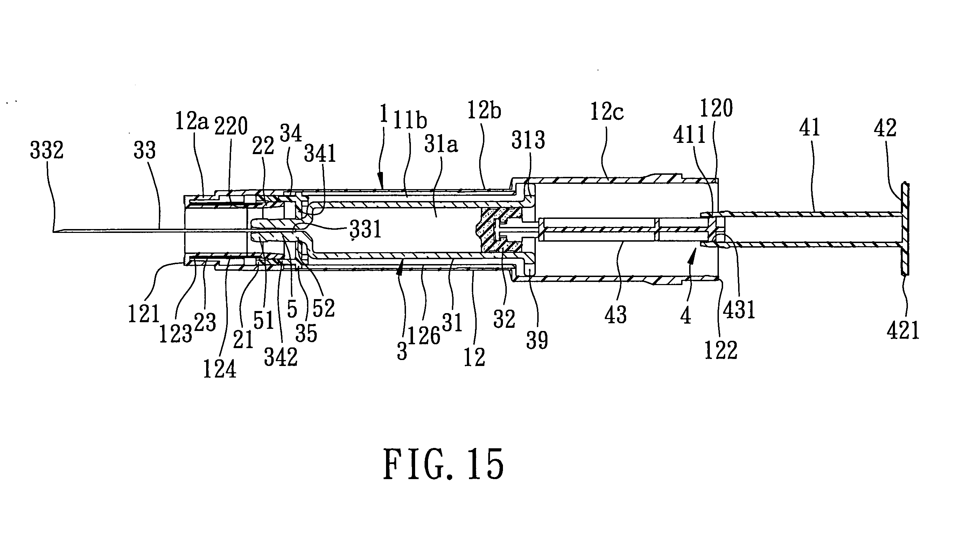

Referring to Figs. 14, 14(A) and 15, the fourth preferred embodiment of a disposable syringe according to this invention is shown to be similar to the first embodiment in construction. In the fourth embodiment, the surrounding barrel wall 12 further includes an enlarged wall portion (12c) which extends from the rear wall portion (12b) to the rear barrel end 122. Thus, the carpule 3 may be a conventional pre-filled syringe which has a larger finger flange 39 that is received within and that is slidable along an inner surface of the enlarged wall portion (12c). In addition, a plurality of ribs (12d) extend radially and inwardly from the enlarged wall portion (12c) such that, when the carrier unit 34 is turned to the locked position, the finger flange 39 is retained by the ribs (12d), thereby retaining the carpule 3 onto the barrel 1 more firmly.

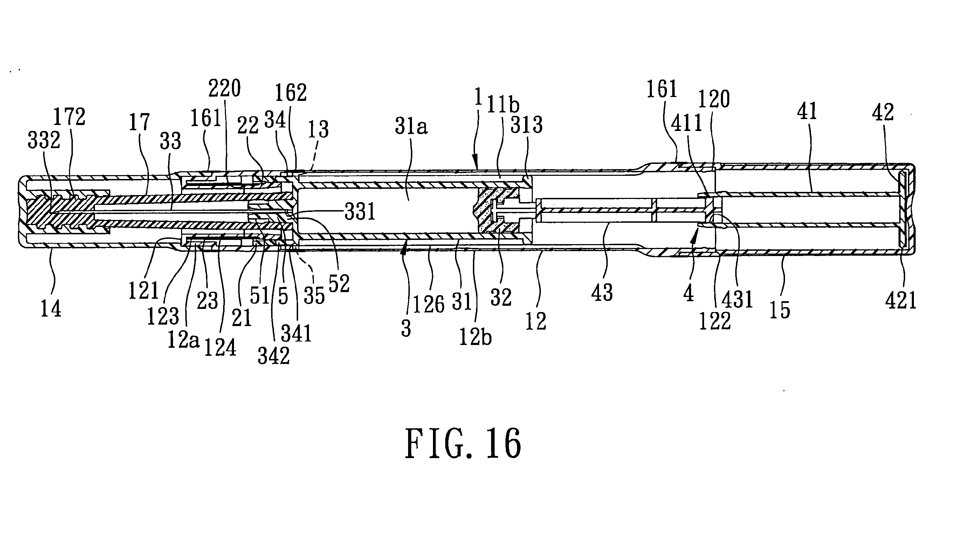

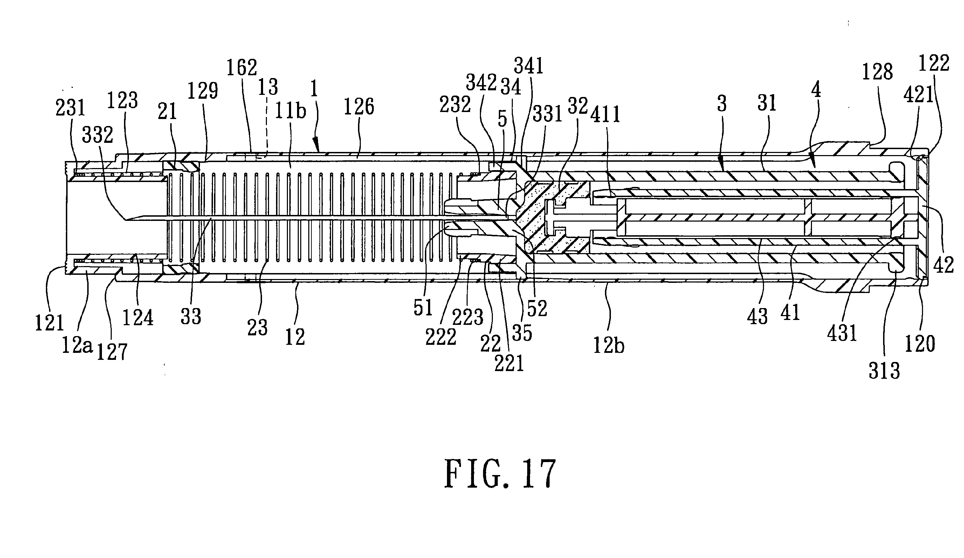

Referring to Figs. 16 and 17, the fifth preferred embodiment of a disposable syringe according to this invention is shown to be similar to the first embodiment in construction. In the fifth embodiment, the tubular wall 31 of the carpule 3 is thermoplastically molded, and the web 341 of the carrier unit 34 is integrally formed with the front tubular edge surface 311.

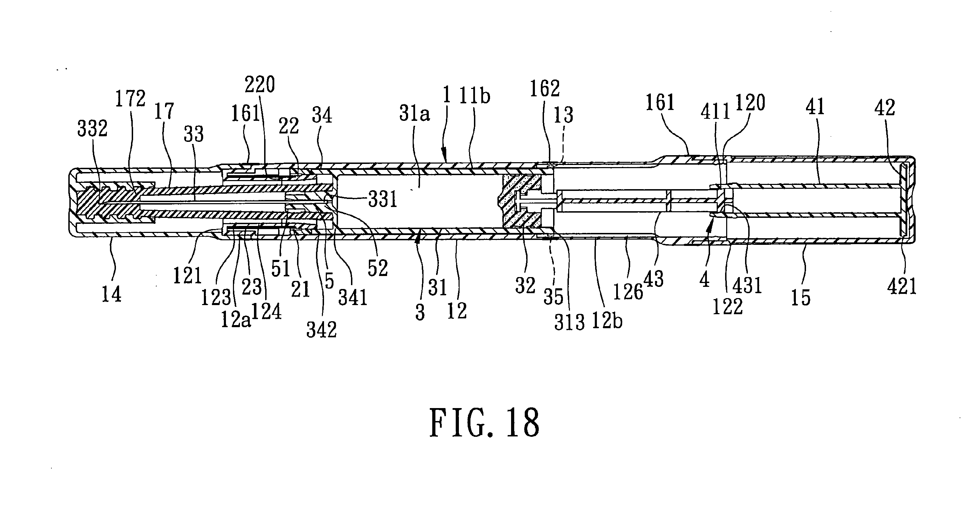

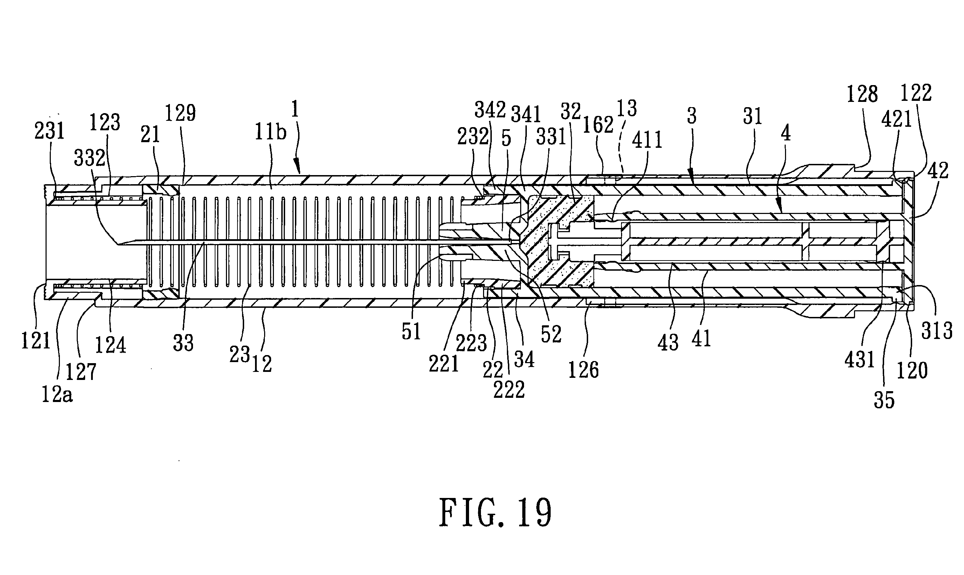

Referring to Figs. 18 and 19, the sixth preferred embodiment of a disposable syringe according to this invention is shown to be similar to the fifth embodiment in construction. In the sixth embodiment, the pegs 35 of the key-and-keywaymechanismare disposed on the rear annular flange 313 of the carpule 3, and the guide slots 126 are formed in the rear wall portion (12b) of the barrel 1 adj acent to the rear barrel end 122 and have a length shorter than those of the first embodiment.

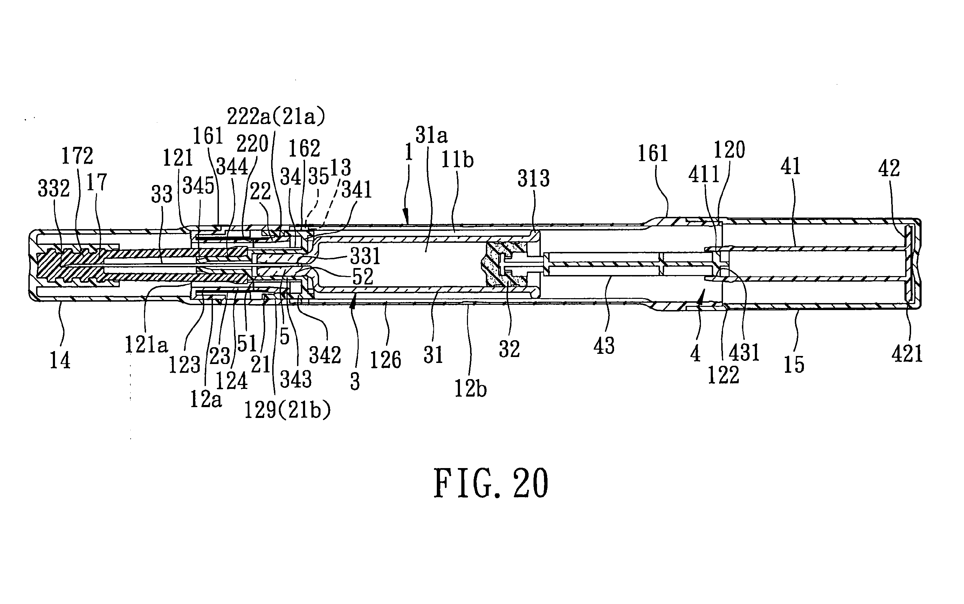

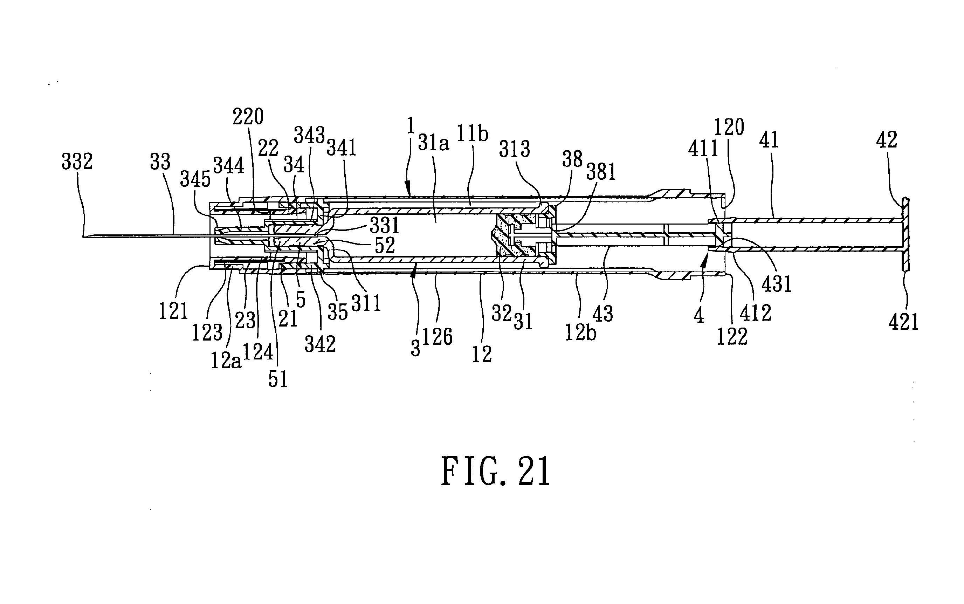

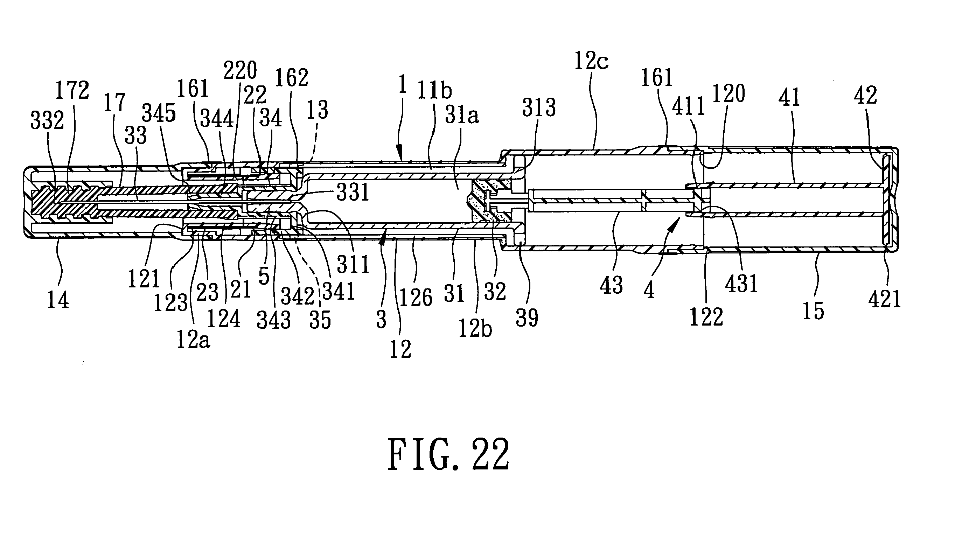

Referring to Figs. 20 to 22, the seventh to ninth preferred embodiments of a disposable syringe according to this invention are shown to be similar to the first, third and fourth embodiments, respectively. In seventh to ninth embodiments, the carrier unit 34 further includes a large-diameter surrounding portion 343 which extends forwardly from the web 341 along the axis and which surrounds the seat unit 5, and a small-diameter surrounding portion 344 which extends forwardly from the large-diameter surrounding portion 343 along the axis to surround the needle cannula 33, and which terminates at a front converging end 345 that is configured to hold the needle cannula 33 steadily in the position of use. Thus, bending of the needle cannula 33 can be prevented during use.

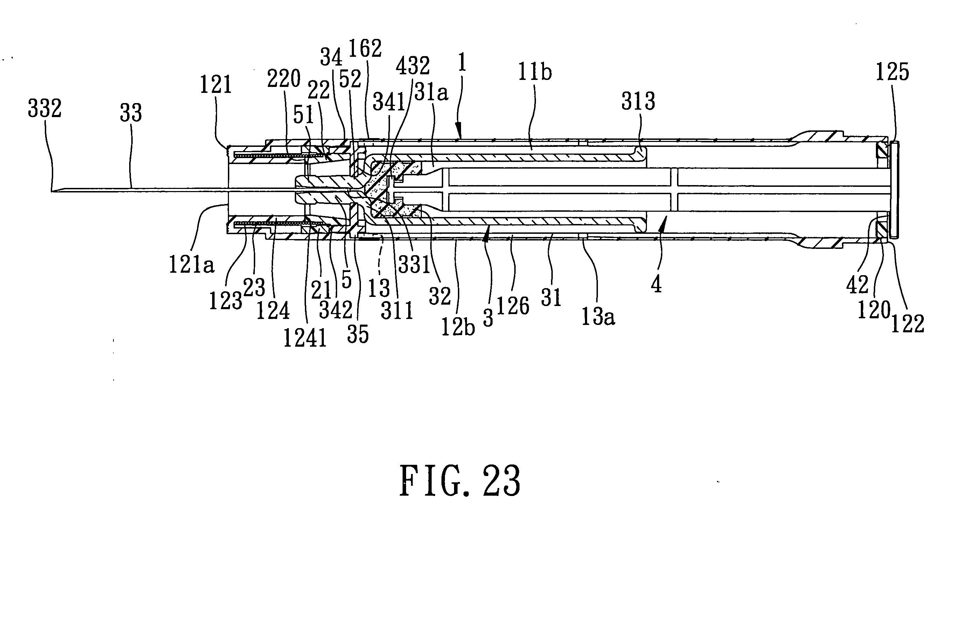

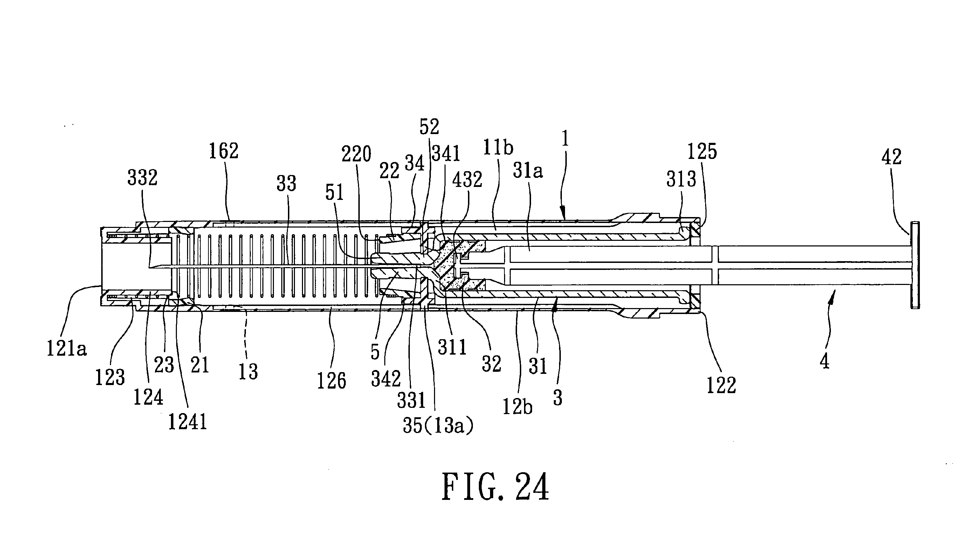

Referring to Figs. 23 and 24, the tenth preferred embodiment of a disposable syringe according to this invention is shown to be similar to the first embodiment. In this embodiment, the plunger 4 is of a single-piece structure, i.e., the push end 432 is integrally formed with the operating end 42. In addition, a tubular barrier 125, such as a ring, extends radially and inwardly from the rear barrel end 122 of the barrel 1. Thus, when the carpule 3 is biased to the disposal position, as shown in Fig. 24, the tubular barrier 125 can guard against incidental movement of the carpule 3 out of the barrel 1. It is noted that the first to ninth embodiments may include such a single-piece plunger 4 instead of one having first and second plunger bodies 43,41.

Furthermore, the retaining mechanism 13 further includes a rear retaining groove (13a) which extends transversely to and which is communicated with the guide slot 126 and which is disposed opposite to the front retaining groove 13. Thus, after the needle cannula 23 is placed in the disposal position, the operator can turn the plunger 4 to bring the peg 35 into the rear retaining groove (13a) so as to guard the carpule 3 against incidental movement.

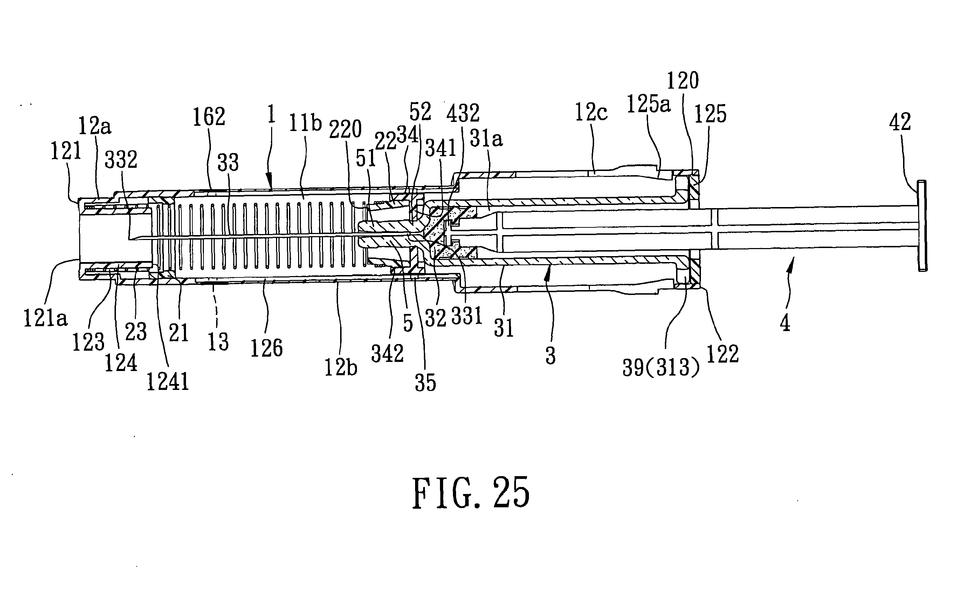

Alternatively, referring to Fig. 25, in the eleventh preferred embodiment of a disposable syringe according to this invention, which is similar to the tenth embodiment, instead of the rear retaining groove (13a), the surrounding barrel wall 12 has an annular elastomeric member (125a) which is disposed forwardly of and which is spaced apart from the tubular barrier 125. Thus, when the carpule 3 is biased to the disposal position, the rear annular flange 313 or 39 of the carpule 3 is moved to slip over the elastomeric member (125a) so as to retain the rear annular flange 313 between the elastomeric member (125a) and the tubular barrier 125, thereby guarding the carpule 3 against forward movement.

As illustrated, the disposable syringe of this invention has the following advantages:
1. Since the carpule 3 can be pre-assembled in the barrel, clinical injection operation by health care workers is simplified and convenient to conduct. In addition, the syringe can be conveniently rendered unreusable after injection in a single operation.
2. By virtue of the air-tight sleeve 17, the air-tightness of the sealed chamber (31a) can be ensured. By virtue of the front and rear protector 14,15 and the sealing strips 161,162, the needle cannula 33 and the plunger 4 can be packed in a sealing and disinfecting manner.
3. By virtue of engagement between the carrier unit 34 and the lunge member 22, the key-and-key way mechanism 35,126, and the retaining mechanism 13, the carpule 3 can be retained firmly in the barrel 1 before use. The injection and needle retraction operations are stable and smooth.
4. Since the operating end 42 of the plunger 4 is retained with the retaining portion 120 of the barrel 1 to guard against a rearward movement of the plunger 4 after the operating end 42 is fully pressed in the barrel 1, reuse of the syringe can be prevented.

## Claims

1. A disposable syringe with a built-in carpule comprising:
a barrel (1) including front and rear barrel ends (121,122) opposite to each other along an axis in an axial direction, and a surrounding barrel wall (12) which interconnects said front and rear barrel ends (121,122), and which includes front and rear wall portions (12a,12b) that are disposed proximate to said front and rear barrel ends (121,122), respectively, and that define front and rear compartments (11a,11b), respectively, said front barrel end (121) defining an insertion opening (121a) which is communicated with said front compartment (11a);
a needle cannula (33) having a tip end (332) which is disposed forwardly of said front barrel end (121) in the position of use, and a communicating end (331) which is opposite to said tip end (332) along the axis;
a seat unit (5) which is configured to secure said communicating end (331) therein, and which has proximate and distal hub segments (51,52) relative to said tip end (332); and
a carpule (3) including
a tubular wall (31) which is configured to be rotatable in said rear compartment (11b) about the axis, and which extends forwardly to terminate at a front tubular edge surface (311) that extends radially to be integrally formed with said distal hub segment (52), and
a seal member (32) disposed to be in fluid-tight engagement with said tubular wall (31) by virtue of a second friction force, said seal member (32) being spaced apart from said front tubular edge surface (311) in the axial direction to define, in cooperation with said tubular wall (31), a sealed chamber (31a) for holding liquid medicament, and being movable forwardly to expel the liquid medicament out of said sealed chamber (31a) to said front tubular edge surface (311), thereby placing said carpule (3) in a used-up position,
**characterized by:**
a holding member (124) which is disposed in said insertion opening (121a), which extends rearwardly to terminate at a limit end (1241), and which is spaced apart from said front wall portion (12a) radially to define an accommodation space (123) therebetween;
a lunge member (22) having
a front lunge end (221) which is disposed to confront and which is permitted to abut against said limit end (1241) in the axial direction, and which has a resting surface (223) confronting said accommodation space (123), and
a ring head (222) which is opposite to said front lunge end (221) in the axial direction, and which has an annular gripped surface (222a) that is spaced apart from said front wall portion (12a) to define a surrounding clearance therebetween;
a ring grip (21) which is disposed in said surrounding clearance, and which has an inner annular gripping surface (21a) and an outer annular gripping surface (21b) to retainingly engage said gripped surface (222a) and said front wall portion (12a), respectively, by virtue of a first friction force, such that in a position of use, the first friction force is of such a magnitude as to guard said front lunge end (221) against movement away from said limit end (1241);
a carrier unit (34) disposed to be rotatable with said carpule (3) about the axis between locked and unlocked positions by an external twisting force, said carrier unit (34) including a push stud (342) which is configured such that, once said carrier unit (34) is in the unlocked position, said push stud (342) is permitted to be pushed forward by a pushing force to move said ring grip (21) forward against the first friction force so as to release said front lunge end (221) from said limit end (1241), thereby placing said lunge member (22) in a released position;
a retaining mechanism (13) disposed to guard said carrier unit (34) against movement away from the locked position;
a key-and-keyway mechanism (35,126) disposed to guide said tubular wall (31) to move along said rear wall portion (12b) in the axial direction once said carrier unit (34) is displaced to the unlocked position;
a plunger (4) having
a push end (432) which is disposed to engage and move said seal member (32) forwardly along said tubular wall (31) against the second friction force so as to bring said carpule (3) to the used-up position,
and
an operating end (42) which is opposite to said push end (432) in the axial direction, and which extends outwardly of said rear barrel end (122) to be subjected to the external twisting force to turn said tubular wall (31) together with said carrier
unit (34) to the unlocked position; and
a biasing member (23) which is disposed in said accommodation space (123) to abut against said resting surface (223) such that, once said lunge member (22) is placed in the released position, said biasing member (23) biases said ring head (222) so as to force said distal hub segment (52) and said carpule (3) towards said rear barrel end (122) to retract said tip end (332) of said needle cannula (33) into said barrel (1), thereby placing said needle cannula (23) in a disposal position.

2. The disposable syringe with a built-in carpule according to Claim 1, **characterized in that** said ring head (222) is configured such that, as a result of squeezing of said ring grip (21) in said surrounding clearance, said ring head (222) acquires an urging force to generate the first friction force between said annular gripped surface (222a) and said inner gripping surface (21a), and between said outer annular gripping surface (21b) and said front wall portion (12a).

3. The disposable syringe with a built-in carpule according to Claim 2, **characterized in that** said carrier unit (34) includes a web (341) which extends radially from said distal hub segment (52) and towards said surrounding barrel wall (12) to terminate at a peripheral portion,
said push stud (342) being formed with said peripheral portion such that, once said lunge member (22) is placed in the released position, said ring head (222) is biased by said biasing member (23) to lunge at said web (341).

4. The disposable syringe with a built-in carpule according to Claim 1, **characterized in that** said plunger (4) includes
a first plunger body (43) having said push end (432) which is disposed to engage and move with said seal member (32), and a first rear end portion (431) opposite to said push end (432), and
a second plunger body (41) having said operating end (42), and a second front end portion (413) which is opposite to said operating end (42), and which is releasably retained with said first rear end portion (431) by a third friction force that is greater than the second friction force so as to permit said seal member (32) to be moved forwardly to thereby place said carpule (3) in the used-up position, and that is greater than the first friction force such that, after said push stud (342) is pushed forward to place said lunge member (22) in the released position, said second plunger body (41) is permitted to move relative to said first plunger body (43), thereby permitting said seat unit (5) and said carpule (3) to be retracted rearwardly to the disposal position.

5. The disposable syringe with a built-in carpule according to Claim 4, **characterized in that** said surrounding barrel wall (12) has a retaining portion (120) adjacent to said rear barrel end (122), said second plunger body (41) being configured such that said operating end (42) is retained with said retaining portion (120) once said seat unit (5) is placed in the released position so as to guard against a rearward movement of said second plunger body (41).

6. The disposable syringe with a built-in carpule according to Claim 1, **characterized in that** said front and rear wall portions (12a,12b) respectively have front and rear shoulder abutments (127,128) which are respectively disposed adjacent to said front and rear barrel ends (121,122) and which respectively face forwardly and rearwardly;
said disposable syringe further comprising:
a front protector (14) which is detachably sleeved on said front wall portion (12a) and which abuts against said front shoulder abutment (127) for shielding said needle cannula (33); and
a rear protector (15) which is detachably sleeved on said rear wall portion (12b) and which abuts against said rear shoulder abutment (128) for shielding said plunger (4).

7. The disposable syringe with a built-in carpule according to Claim 6, **further characterized by** front and rear sealing strips (161) which are disposed to peelably adhere said front protector (14) to said front wall portion (12a), and to peelably adhere said rear protector (15) to said rear wall portion (12b), respectively.

8. The disposable syringe with a built-in carpule according to Claim 1, **further characterized by** an air-tight sleeve (17) which is removably sleeved on said seat unit (5) for maintaining air-tightness of said sealed chamber (31a).

9. The disposable syringe with a built-in carpule according to Claim 8, **characterized in that** said air-tight sleeve (17) is disposed to shield said needle cannula (33), and has an elastomeric front end (172) such that, when said air-tight sleeve (17) is sleeved on said seat unit (5), said tip end (332) of said needle cannula (33) is trapped in said elastomeric front end (172).

10. The disposable syringe with a built-in carpule according to Claim 1, **characterized in that** said push stud (342) is disposed to be in frictional engagement with and is
permitted to rotate relative to said ring head (222) in the position of use, said carrier unit (34) being configured such that, when said lunge member (22) is placed in the released position by means of a forward displacement of said push stud (342) to move said ring grip (21) forward, said push stud (342) is in a frictional engagement with said gripped surface (222a) of said ring head (222) for facilitating displacement of said needle cannula (23) to the disposal position.

11. The disposable syringe with a built-in carpule according to Claim 1, **characterized in that** said front lunge end (221) of said lunge member (22) is spaced apart from said limit end (1241) by a small clearance (220) in the position of use, such that said clearance (220) serves as a triggering space (220) for facilitating a rearward movement of said lunge member (22) after said lunge member (22) is moved forwardly with said ring grip (21) to bring said front lunge end (221) into abutment against said limit end (1241).

12. The disposable syringe with a built-in carpule according to Claim 3, **characterized in that** said carrier unit (34) includes a large-diameter surrounding portion (343) which extends forwardly from said web (341) along the axis and which surrounds said seat unit (5), and a small-diameter surrounding portion (344) which extends forwardly from said large-diameter surrounding portion (343) along the axis to surround said needle cannula (33), and which terminates at a front converging end (345) that is configured to hold said needle cannula (33) steadily in the position of use.

13. The disposable syringe with a built-in carpule according to Claim 3, **characterized in that** said web (341) of said carrier unit (34) is disposed to be press-fitted to said seat unit (5).

14. The disposable syringe with a built-in carpule according to Claim 1, **characterized in that** said push end (432) of said plunger (4) is integrally formed with said operating end (42), said disposable syringe further comprising a tubular barrier (125) extending radially and inwardly from said rear barrel end (122) so as to guard against incidental movement of said carpule (3) out of said barrel (1) when said carpule (3) is biased to the disposal position.

15. The disposable syringe with a built-in carpule according to Claim 1, **characterized in that** said surrounding barrel wall (12) includes an enlarged wall portion (12c) which extends from said rear wall portion (12b) to said rear barrel end (122), and a plurality of ribs (12d) which extend radially and inwardly from the enlarged wall portion (12c), said tubular wall (31) of said carpule (3) having a finger flange (39) which is received within and which is slidable along an inner surface of said enlarged wall portion (12c), and which is configured such that, when said carrier unit (34) is turned to the locked position, said finger flange (39) is retained by said ribs (12d), thereby firmly retaining said carpule (3) onto said barrel (1).
